# FASCICULE DE BREVET EUROPEEN

(11) **EP 3 700 410 B1**
(45) Date de publication et mention de la délivrance du brevet: **26.11.2025**
(21) Numéro de dépôt: 18808423.0
(22) Date de dépôt: 24.10.2018
(51) Int. Cl.: A61B 5/00, A61B 5/0275

(54) **PROCÉDÉ ET DISPOSITIF DE SUIVI DE LA FLUORESCENCE ÉMISE À LA SURFACE D'UN TISSU BIOLOGIQUE**
VERFAHREN UND VORRICHTUNG ZUR ÜBERWACHUNG DER AN DER OBERFLÄCHE VON BIOLOGISCHEM GEWEBE EMITTIERTEN FLUORESZENZ
METHOD AND DEVICE FOR MONITORING THE FLUORESCENCE EMITTED AT THE SURFACE OF A BIOLOGICAL TISSUE

(30) Priorité: 26.10.2017 FR 1760111
(43) Date de publication de la demande: 02.09.2020
(73) Titulaire: Fluoptics, 38040 Grenoble (FR)
(72) Inventeur: DAURES, Anthony, 92110 Clichy (FR); RIZO, Philippe, 38700 La Tronche (FR)
(74) Mandataire: Santarelli
(86) Numéro de dépôt international: PCT/FR2018/052639
(87) Numéro de publication internationale: WO 2019/081849

(56) Documents cités:
- US-A1- 2009 238 435
- US-B2- 8 743 241
- A. J. STRONG ET AL: "The Use of In Vivo Fluorescence Image Sequences to Indicate the Occurrence and Propagation of Transient Focal Depolarizations in Cerebral Ischemia", JOURNAL OF CEREBRAL BLOOD FLOW & METABOLISM, vol. 16, no. 3, 1 May 1996 (1996-05-01), US, pages 367 - 377, XP055485984, ISSN: 0271-678X, DOI: 10.1097/00004647-199605000-00003
- LICHAO LIAN ET AL: "High-dynamic-range fluorescence molecular tomography for imaging of fluorescent targets with large concentration differences", OPTICS EXPRESS, vol. 24, no. 17, 19 August 2016 (2016-08-19), pages 19920, XP055485338, DOI: 10.1364/OE.24.019920
- C. VINEGONI ET AL: "Real-time high dynamic range laser scanning microscopy", NATURE COMMUNICATIONS, vol. 7, 1 April 2016 (2016-04-01), pages 11077, XP055485331, DOI: 10.1038/ncomms11077
- FLORA HUI ET AL: "Quantitative Spatial and Temporal Analysis of Fluorescein Angiography Dynamics in the Eye", PLOS ONE, vol. 9, no. 11, 3 November 2014 (2014-11-03), XP055295081, DOI: 10.1371/journal.pone.0111330
- AHMED S FAHMY ET AL: "Segmentation of Choroidal Neovascularization lesions in fluorescein angiograms using parametric modeling of the intensity variation", 2011 8TH IEEE INTERNATIONAL SYMPOSIUM ON BIOMEDICAL IMAGING: FROM NANO TO MACRO (ISBI 2011), IEEE, UNITED STATES, 30 March 2011 (2011-03-30), pages 665 - 668, XP031944626, ISBN: 978-1-4244-4127-3, DOI: 10.1109/ISBI.2011.5872494

## Description

L'invention concerne le domaine de l'imagerie médicale. Plus particulièrement, l'invention concerne un procédé et un dispositif de suivi de la fluorescence dans un tissu biologique.

Un exemple de procédé d'imagerie médicale basée sur la fluorescence est par exemple décrit dans le document « The Use of In Vivo Fluorescence Image Sequences to Indicate the Occurrence and Propagation of Transient Focal Depolarizations in Cerebral Ischemia », A. J. Strong, et Al., Journal of cerebral blood flow & metabolism, vol. 16, no.3, 1 May 1996, pages 367-377. Un système d'imagerie médicale basée sur la fluorescence d'un marqueur est décrit par exemple dans le document FR2989876A2. Le documents US 8 743 241 B2 (LESIAK ET AL) et US 2009/238435 A1 (SHIELDS KEVIN ET AL) présentent des procédés d'imagerie de fluorescence HDR (High Dynamic Range).

L'imagerie de fluorescence permet de donner une information peropératoire quant à la perfusion dans des tissus biologiques, notamment humains. Pour certaines indications médicales, il peut être intéressant d'identifier et de localiser des zones du tissu biologique dans lesquelles le signal de fluorescence apparaît en premier, celles pour lesquelles la vitesse de montée et de descente du signal fluorescent (donc la pente du signal, c'est-à-dire la vitesse d'augmentation et de diminution de l'intensité du signal) est plus ou moins importante, ainsi que l'amplitude du signal de fluorescence dans les différentes zones (qui représente le niveau de perfusion dans celles-ci). En effet, ceci peut permettre d'évaluer la perfusion globale du tissu et d'identifier d'éventuels problèmes veineux ou artériels (chirurgie plastique, plaies et cicatrisations, ...).

Pour ces indications il est nécessaire de bien appréhender la dynamique du signal de fluorescence.

Des procédés ont été proposés dans lesquels des informations relatives à la dynamique de la perfusion peuvent être affichées et visualisées. Cependant, ceux-ci nécessitent l'intervention et l'expertise d'un médecin ou chirurgien pour définir des zones de référence dans les tissus observés (par exemple, le chirurgien doit pointer et désigner le tissu sain sur l'image, pour qu'une normalisation du signal soit réalisée par rapport à ce tissu). En outre, les calculs de pente et d'amplitude du signal de fluorescence peuvent être biaisés du fait d'artéfacts liés à :
- un changement de lumière dans l'environnement de la salle ou
- un déplacement de la zone d'intérêt au cours du temps (mouvements du patient).

De même, fournir une représentation graphique (par exemple une courbe de montée du signal de fluorescence) sans apporter d'information visuelle, notamment relative au contexte, constitue un risque de mauvaise interprétation des résultats.

L'invention a pour but de fournir des moyens permettant d'améliorer, au moins partiellement la connaissance de la dynamique de perfusion et de palier, également au moins partiellement, certains des inconvénients des techniques de l'art antérieur.

Ce but est au moins en partie atteint avec un procédé de suivi de la diffusion dans le temps d'un marqueur fluorescent au sein d'un tissu biologique, tel que défini par la revendication 1.

Dans ce document on appelle « signal de fluorescence », la valeur relative du signal mesuré sur un pixel, à l'aide de la caméra, ce signal étant représentatif de l'intensité de l'émission de fluorescence en un point de la zone du tissu (correspondant à ce pixel).

Grâce à l'invention, il est possible de visualiser directement sur une image du tissu biologique observé (sur l'image d'un pied par exemple), le résultat d'une opération de calcul permettant la comparaison automatique, pixel par pixel, de deux images représentatives de la diffusion, observée à au moins deux temps successifs, du marqueur fluorescent. L'image résultant de cette opération de calcul fait apparaitre la variation entre deux temps de mesure, du signal de fluorescence localement (mais sur l'ensemble de la zone observée du tissu, pas seulement sur une zone restreinte ou sur quelques pixels, donc sur l'ensemble d'un pied par exemple). De cette façon, il est possible de suivre la dynamique de la perfusion en visualisant comment cette variation évolue dans le temps et localement (en visualisant la variation du signal sur chaque pixel mais parmi l'ensemble des pixels de l'image). Autrement dit, il est possible grâce à l'invention de visualiser la dynamique de la perfusion dans une zone et dans l'environnement autour de cette zone. On peut par exemple ainsi voir si un signal augmente autour d'une certaine zone, alors qu'il n'augmente pas dans cette zone, qu'une artère vascularisant cette zone est bouchée.

Les images obtenues avec le procédé selon l'invention peuvent être interprétées par des techniciens dans un premier temps (par exemple pour des applications à l'analyse d'éventuelles plaies chroniques - « wound care » en anglais), un médecin intervenant éventuellement si un problème nécessitant son expertise est détecté par le technicien. Et si un médecin doit intervenir, celui-ci peut le faire plus rapidement.

Le procédé selon l'invention comporte éventuellement l'une ou l'autre des caractéristiques correspondant aux revendications 2 à 5 considérées indépendamment les unes des autres ou en combinaison d'une ou plusieurs autres.

L'invention concerne également un dispositif de suivi de la fluorescence émise à la surface du tissu biologique comprenant :
- une source d'excitation adaptée pour émettre un rayonnement d'excitation d'un marqueur de fluorescence,
- une caméra comprenant un capteur de la lumière de fluorescence émise à la surface du tissu biologique, sous l'effet du rayonnement d'excitation,
- un ordinateur pour enregistrer et stocker des images de fluorescence capturées par la caméra, et pour réaliser un traitement des images de fluorescence, et
- un écran pour afficher des images résultant du traitement des images de fluorescence par l'ordinateur, l'ordinateur comprenant des moyens de calcul pour traiter des images de fluorescence à l'aide d'un logiciel pour la mise en œuvre du procédé tel que mentionné ci-dessus.

Le dispositif selon l'invention comporte éventuellement une source de lumière éclairant dans une bande spectrale dans laquelle un marqueur est sensible et dans laquelle la fluorescence de ce marqueur est donc excitée. Le capteur de la lumière de fluorescence n'étant pas sensible dans cette bande spectrale d'excitation, mais étant sensible dans la gamme de longueurs d'ondes correspondant à l'émission de la fluorescence du marqueur.

D'autres caractéristiques et avantages de l'invention apparaîtront à la lecture de la description détaillée qui suit, ainsi que sur les dessins annexés. Sur ces dessins :
- la figure 1 représente schématiquement un mode de réalisation d'un dispositif selon l'invention ;

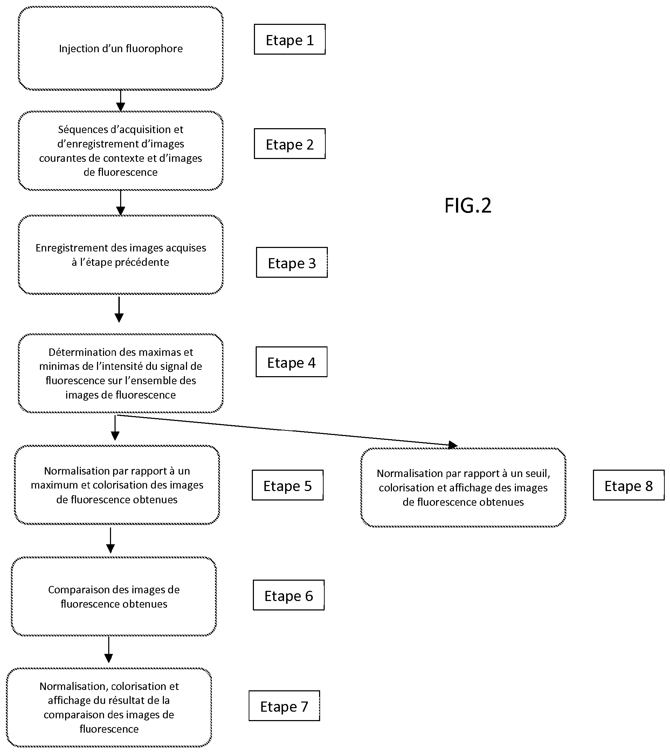

- la figure 2 représente schématiquement un ensemble d'étapes successives correspondant à un exemple de mise en œuvre du procédé selon l'invention ;
- la figure 3 représente schématiquement l'évolution au cours du temps de l'intensité moyenne du signal de fluorescence sur une zone de tissu biologique ;
- la figure 4 représente schématiquement la dynamique du signal de fluorescence sur une zone de tissu biologique ;
- la figure 5 représente schématiquement l'évolution au cours du temps de l'intensité moyenne du signal de fluorescence sur une zone de tissu biologique, normalisée par un seuil qui correspond à un seuil arbitraire supposé correspondre à un pied sain ;
- la figure 6 représente schématiquement une synthèse des résultats du type de ceux représentés sur les figures 3, 4 et 5, mais pour des temps d'acquisition correspondant à des intervalles de temps inégaux.

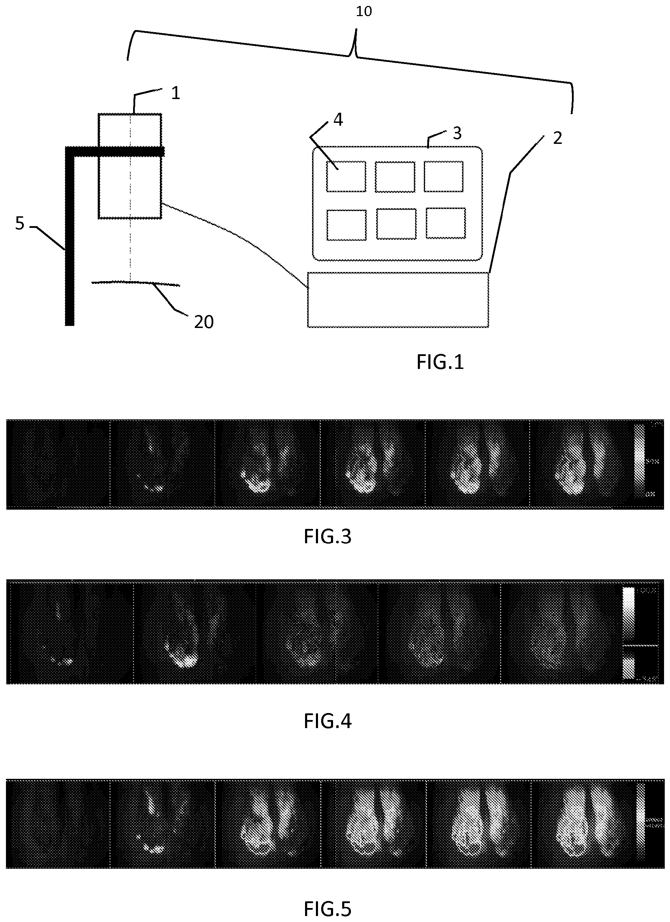

Un exemple de mode de réalisation d'un dispositif 10 de suivi de la fluorescence émise à la surface du tissu biologique 20 est représenté sur la figure 1.

Ce dispositif 10 comporte une sonde 1 avec une caméra dite « de fluorescence » pour capturer des images de fluorescence (dans le proche infra-rouge ou plus généralement dans des longueurs d'ondes détectées par cette caméra « de fluorescence »).

Autrement dit, cette caméra est dotée d'au moins un capteur adapté pour capter des images dans le proche infra-rouge ou plus généralement dans les longueurs d'ondes émises par des marqueurs fluorescents. Cette caméra permet donc de réaliser une image de la lumière de fluorescence émise par le fluorophore à la surface d'une zone de tissu biologique 20.

Dans ce document, on nomme « image de fluorescence », une image du signal de fluorescence émis à la surface du tissu biologique 20 à observer, capturée à l'aide d'une caméra « de fluorescence » et on nomme « image courante », une image extraite (directement, sans intégration ou sommation avec une ou plusieurs autres images) d'une vidéo réalisée à l'aide d'une caméra de la sonde 1. L'« image courante » peut être réalisée, par exemple, en éclairant le tissu biologique 20 à observer à l'aide de diodes électroluminescentes émettant dans le proche infra-rouge (ou plus généralement dans les longueurs d'ondes détectées par la caméra « de fluorescence »), et /ou en éclairant le tissu biologique 20 à observer à l'aide d'une source lumineuse adaptée pour exciter le ou les marqueurs fluorescents.

Selon une variante, la sonde 1 comporte une première caméra de fluorescence pour capturer des images de fluorescence (par exemple dans le proche infra-rouge) et une deuxième caméra pour capturer des images dans le visible. Dans ce cas, des « images courantes » peuvent être réalisées soit dans la gamme de longueurs d'ondes détectées par la caméra de fluorescence, soit dans le visible.

Le dispositif 10 comporte également un ordinateur 2 pour enregistrer et stocker les images capturées par chaque caméra, ainsi que pour réaliser un traitement des images de fluorescence et éventuellement des images courantes.

Le dispositif 10 comporte aussi des moyens de visualisation et d'affichage 3 (écran) d'images 4 après traitement.

La sonde 1 est par exemple connectée à l'ordinateur 2.

La sonde 1 comprend également une source d'excitation (laser par exemple) adaptée pour émettre un rayonnement d'excitation d'un marqueur de fluorescence ou fluorophore.

La caméra de fluorescence comprend un capteur sensible à la lumière de fluorescence émise par le fluorophore à la surface de cette zone de tissu biologique 20.

La sonde 1 est avantageusement maintenue, à l'aide d'un bras de support 5, de manière stable et à une distance constante de la scène comprenant la zone de tissu biologique 20 à observer et étudier (il peut toutefois y avoir un léger déplacement de la scène du fait notamment de la respiration du patient).

Un traceur fluorescent, ou fluorophore, est injecté en intra-veineuse. Le signal d'émission du fluorophore est capté par la caméra « de fluorescence » de la sonde 1 et est enregistré au cours du temps en laissant la source d'excitation allumée.

Des images de fluorescence sont enregistrées à partir d'un temps T₁ supérieur ou égal à un temps T₀ correspondant au temps d'injection du fluorophore en intraveineuse. On poursuit l'enregistrement jusqu'à un temps final T_{F} considéré comme suffisamment long pour obtenir la majeure partie de la dynamique de perfusion.

Un logiciel contrôle l'acquisition de séquences d'images courantes et de fluorescence. L'intervalle de temps *Δt* entre chaque séquence peut être prédéterminé. Par exemple, cet intervalle de temps *Δt* est régulier et correspond à vingt secondes. Donc, dans ce cas, toutes les vingt secondes par exemple, le logiciel commande l'acquisition d'une séquence d'images courante(s) et de fluorescence. Afin d'augmenter la précision des mesures du signal de fluorescence correspondant à une séquence, une séquence peut être composée d'images de fluorescence acquises avec des temps d'exposition différents et/ou des gains différents. L'intervalle de temps entre la première et la dernière images de chacune de ces séquences est choisi suffisamment court pour pouvoir considérer que le signal de fluorescence est resté statique par rapport à l'ensemble de la dynamique de la perfusion. Autrement dit, le temps d'acquisition sur lequel s'écoule chaque séquence est suffisamment court pour être considéré comme négligeable par rapport à la vitesse d'évolution du signal mesuré.

Une séquence se compose, par exemple, des sous-séquences ou séries suivantes :
1) N images de fluorescence avec un temps d'exposition X secondes,
2) M images de fluorescence avec un temps d'exposition 2*X secondes,
3) P images de fluorescence avec un temps d'exposition 3*X secondes,
4) Une image courante réalisée avec la source d'excitation laser éteinte et un éclairage comprenant des diodes électroluminescentes émettant dans l'infra-rouge allumé, pour obtenir une image courante du fond, I_{C}, seule, sans fluorescence. On notera que pour cette pour l'étape 4), selon une variante, la source d'excitation reste allumée, mais on fait un traitement ensuite pour retrouver une image courante en retranchant une image obtenue avec seulement la source d'excitation allumée, d'une image obtenue avec la source d'excitation ainsi que l'éclairage comprenant des diodes électroluminescentes, tous deux allumés. Autrement dit, la source d'excitation reste toujours allumée, et on se ramène à une image sans source d'excitation en soustrayant une image (ou une moyenne d'images) sans éclairage par diodes électroluminescente, à l'image (ou à une moyenne d'images) où celles-ci sont allumées. On notera également que N, M et P peuvent être des nombres entiers égaux entre eux, ou différents deux à deux, etc.

On notera également qu'au lieu de faire faire varier les temps d'exposition aux étapes 1), 2) et 3), on aurait pu faire varier les gains.

Augmenter le temps d'exposition - sous-séquences 2) et 3) ci-dessus - permet de faire ressortir du signal noyé dans le bruit pour l'exposition correspondant à la sous-séquence 1). Avec une caméra linéaire, augmenter le temps d'exposition par deux revient à augmenter le niveau de signal moyen par deux. Donc, en utilisant ce type de caméra, on peut aisément ramener toutes les images à des niveaux d'expositions identiques, même avec des temps de pose différents. Ce type de séquence permet d'augmenter la précision de la mesure du signal de fluorescence et d'atténuer le bruit (effet moyenneur sur le bruit Poissonnien).

Plus particulièrement, les niveaux de gris obtenus pour chaque temps d'exposition sont sommés pixel par pixel. Préalablement à la sommation, les images sont recalées entre elles et les pixels correspondant à une saturation du signal sont éliminés. Ce recalage des images entre elles peut se faire à l'aide d'un algorithme standard de recalage d'images. Cependant, on privilégie les algorithmes de recalage à au moins six degrés de liberté (rotations + translations), avec des points singuliers, des flux optiques, ...

Le temps d'exposition total par pixel est comptabilisé (sans prendre en compte les temps d'exposition correspondant à une saturation pour le pixel considéré, ou en prenant en compte les temps d'exposition pour ces pixels, mais en remplaçant la valeur du signal pour ces pixels par une extrapolation à partir de valeurs obtenues sur des images dans lesquelles le signal n'est pas saturé). La somme des niveaux de gris par pixel est divisée par le temps d'exposition total correspondant à ce pixel, de manière à obtenir ensuite des images normalisées sur un même temps d'exposition. On notera, que selon des variantes, on peut appliquer des pondérations ou réaliser une conversion non-linéaire des couleurs avec des tables de couleurs. Ce type de variantes peut être utile notamment pour gérer des variations de signal ayant une dynamique importante. Ceci revient alors par exemple à augmenter un peu les faibles signaux et à réduire les signaux les plus forts, afin que le signal puisse être affiché sur toute son amplitude de variation sans générer de saturation ou de sous-exposition.

Pour les images ainsi obtenues, la précision, notamment dans les faibles signaux, est plus importante. En effet, comme indiqué ci-dessus, augmenter le temps d'exposition permet de faire ressortir le signal par rapport au bruit. Ceci est plus particulièrement le cas avec une caméra « de fluorescence » munie d'un capteur de type « dispositif à couplage de charge » (« Charge-Coupled Device » ou CCD en anglais). Ainsi les signaux faibles ressortent d'autant mieux du bruit que les temps d'exposition sont importants. Le logiciel permet de moyenner le bruit Poissonnien dans l'image (et donc de le réduire).

Un exemple plus détaillé de mode de mise en œuvre du procédé selon l'invention est décrit ci-dessous.

Les temps Tᵢ pour lesquels il faut capturer l'information de fluorescence (avec i allant de 0 à F ; F étant le nombre d'images sur lesquelles le praticien souhaite réaliser l'étude ; pour l'exemple décrit ici F=6), sont saisis comme paramètres dans le logiciel.

Cette acquisition comporte alors par exemple, les étapes suivantes :
Etape 1 : Un chronomètre est mis en marche. A T₀, le praticien injecte de l'ICG (Indocyanine-green) et lance une acquisition d'images à l'aide du logiciel.
Etape 2 : A un temps T₁ postérieur ou égal à T₀, le logiciel déclenche les opérations suivantes :
   ∘ Acquisition et enregistrement d'une image courante du contexte, ou image de fond I_{C1}; pendant cette acquisition la source d'excitation de la fluorescence est éteinte ; comme mentionné plus haut, cette acquisition de l'image courante de fond I_{C1}, selon le type de sonde, peut être réalisée par exemple à l'aide soit d'une caméra munie d'un capteur de lumière dans le visible, soit d'une caméra munie d'un capteur de lumière dans le proche infra-rouge (dans ce dernier cas, la source d'excitation laser est préférentiellement éteinte et un éclairage comprenant des diodes électroluminescentes émettant dans l'infra-rouge est allumé) ;
   ∘ Acquisition et enregistrement d'une séquence d'images de fluorescence (la source d'excitation laser est allumée et l'éclairage comprenant les diodes électroluminescentes émettant dans l'infra-rouge est préférentiellement éteint, s'il ne l'est pas on procède comme indiqué ci-dessus) ; cette acquisition comme indiqué ci-dessus peut éventuellement comprendre des séries d'images correspondant à des temps d'exposition différents (ex : une série de N images à un temps d'exposition X, puis une ou plusieurs séries de M images correspondant à un ou des temps d'exposition Y plus grand que X (ou des gains différents), afin de réaliser une image en grande gamme dynamique (« High dynamic range » ou HDR en anglais) et profiter d'un meilleur rapport signal sur bruit.
   ∘ Calcul d'une image de fluorescence I₁ résultant de la somme des images de fluorescence traitées comme indiqué ci-dessus (recalage des images entre elles, suppression ou remplacement des pixels saturés, normalisation pour un temps d'exposition donné). Au lieu de sommer des images, il est possible, selon différentes variantes de réaliser un cumul d'images, avec des opérations linéaires ou non, des pondérations ou non, etc... ;

Cette image I₁ représente donc un « arrêt sur image » du niveau de perfusion au temps T₁.

Les opérations de l'étape 2 sont répétées lorsque chaque temps Tᵢ (avec cette fois i= 2 à F) est atteint, jusqu'à finir par l'acquisition au temps T_{F}.

Etape 3 : A la fin, après la dernière acquisition au temps T_{F} les différents niveaux de fluorescence correspondant à des images de fluorescence, Iᵢ, ainsi que les images courantes de fond I_{Ci} sont enregistrées en mémoire dans l'ordinateur 2 (alternativement, des images sont enregistrées en mémoire dans l'ordinateur en cours d'acquisition).

Etape 4 : Le logiciel détermine alors une valeur maximale de l'intensité du signal de fluorescence pour l'ensemble des images de fluorescence Iᵢ. Cette valeur maximale de l'intensité peut être choisie en utilisant un x^{ème} percentile, x% de la valeur maximum et/ou en lissant les images de fluorescence Iᵢ pour éviter des artéfacts ponctuels sur chacune de ces images de fluorescence Iᵢ.

Etape 5 : Le logiciel normalise ensuite chaque image de fluorescence Iᵢ par rapport au maximum déterminé à l'étape précédente. Puis, le logiciel colorise les images de fluorescence Iᵢ ainsi normalisées à l'aide d'une table de conversion des couleurs spécifique. Puis le logiciel superpose ce résultat sur l'image courante de contexte correspondant à ce temps, en laissant apparaitre en niveaux de gris les pixels de l'image courante de contexte qui ne présentent pas de fluorescence. Un exemple d'une série d'images de fluorescence Iᵢ obtenues, à l'aide du procédé ci-dessus, est représenté sur la figure 3. Une telle série d'images de fluorescence Iᵢ correspond à une étude de la vascularisation d'un pied sain.

Etape 6 : Cependant, il est aussi possible d'encore mieux faire ressortir la dynamique des signaux de fluorescence au cours du temps. Pour cela, le logiciel offre la possibilité de comparer entre eux les niveaux d'intensités d'images de fluorescence Iᵢ successives dans le temps. Ainsi, l'intensité de l'image de fluorescence Iᵢ₊₁ prise à Tᵢ₊₁ peut être soustraite (après recalage), pixel par pixel, à l'intensité de l'image de fluorescence Iᵢ prise à Tᵢ. Cette différence permet de faire ressortir ce qui s'est passé entre le temps Tᵢ et le temps Tᵢ₊₁.

On peut noter que la simple soustraction des intensités du signal de fluorescence associé à chaque pixel n'est pas la seule manière de mettre en évidence la dynamique d'un signal. D'une manière générale, le logiciel peut calculer la différence entre le carré des intensités, ou encore la différence entre des logarithmes des intensités, etc. ou n'importe quelle distance au sens mathématique du terme, et plus particulièrement une distance algébrique, entre deux images de fluorescence successives. Ainsi, le calcul sur lequel est basée l'opération de comparaison comprend au moins une opération choisie parmi les opérations suivantes : une soustraction entre des valeurs d'un signal représentatif de l'intensité de l'émission de fluorescence, un calcul de norme d'une grandeur représentée par des valeurs d'un signal représentatif de l'intensité de l'émission de fluorescence, un calcul de distance algébrique entre des valeurs d'un signal représentatif de l'intensité de l'émission de fluorescence, et une opération logique « or » ou « nor » ou « xor » sur des valeurs d'un signal représentatif de l'intensité de l'émission de fluorescence. Ces opérations logiques peuvent en effet permettre de mettre en évidence les phénomènes suivants :
- Il n'y a de signal de fluorescence associé à un pixel ou à un groupe de pixels, ni sur l'image résultant de la première séquence, ni sur l'image résultant de la deuxième séquence d'acquisition (opération « NOR »): ceci fait apparaitre des zones qui ne sont jamais vascularisées.
- Il y a un signal de fluorescence associé à un pixel ou à un groupe de pixels, soit sur l'image résultant de la première séquence, soit sur l'image résultant de la deuxième séquence d'acquisition (opération « OR »): ceci fait apparaitre des zones qui ne sont vascularisées à des temps différents.
- Il y a un signal de fluorescence associé à un pixel ou à un groupe de pixels, que sur l'image résultant de la première séquence, ou sur l'image résultant de la deuxième séquence d'acquisition (opération « XOR »): ceci fait apparaitre des zones qui ne sont vascularisées qu'au cours de l'une des séquences.

Pour pouvoir comparer des images de fluorescence Iᵢ entre elles, il est nécessaire que les images de fluorescence Iᵢ soient recalées entre elles afin que les pixels se correspondent entre les différentes images de fluorescence Iᵢ. Ainsi, la différence entre les images (ou plus généralement l'opération qui permet de comparer les images entre elles) peut être calculée pixel par pixel.

On notera que ces opérations de comparaison ne font pas intervenir dans le calcul une image de référence (« base line image ») acquise par exemple avant l'apparition de la fluorescence, et qui serait soustraite notamment à chaque image résultant d'une séquence. En effet, notamment lors d'une opération de comparaison de deux images résultant de deux séquences, l'utilisation d'une telle image de référence est inutile puisque l'information correspondant disparait par soustraction, dans l'opération de comparaison.

Etape 7 : Une fois ce calcul réalisé par le logiciel, pour des couples d'images successives, le logiciel détermine les valeurs maximales et minimales ainsi obtenues par le calcul de la distance (au sens indiqué ci-dessus) entre les images de fluorescence successives. Ces valeurs maximale et minimale peuvent éventuellement être positives ou négatives. Les pixels auxquels est attachée une valeur négative de la distance calculée précédemment correspondent à des zones où le signal de fluorescence est plus faible à l'instant Tᵢ₊₁ qu'à l'instant Tᵢ et à l'inverse une valeur positive correspond à une hausse de la fluorescence à cet endroit, pendant l'intervalle de temps correspondant.

Le logiciel normalise donc les pixels positifs des images obtenues par le calcul de distance précédent, par le maximum obtenu sur l'ensemble des calculs effectués sur les images de fluorescence d'une séquence de T₁ à T_{F}. De même, le logiciel normalise les pixels négatifs des images obtenues par le calcul de distance précédent, par le minimum obtenu sur l'ensemble des calculs effectués sur les images d'une séquence de T₁ à T_{F}.

Le logiciel colorise les images ainsi normalisées avec une fausse couleur spécifique pour les pixels positifs (par exemple des couleurs chaudes, du jaune au rouge) et une fausse couleur spécifique pour les pixels négatifs (par exemple des couleurs froides du bleu au violet,). Le logiciel permet d'afficher le résultat obtenu (voir figure 4), en superposant chaque image colorisée sur l'image courante de contexte correspondante. Une telle présentation des résultats permet de donner une information visuelle rapide sur les zones correspondant à un signal qui augmente, à partir de quel moment ce signal augmente dans les zones en question (donc sous quel délai), sur quelle période de temps, etc. De même, ce type de présentation des résultats permet de donner une information visuelle rapide sur les zones correspondant à un signal qui diminue, à partir de quel moment il diminue, sur quelle période de temps, etc.

Ce type d'affichage peut notamment permettre de visualiser des problèmes artériels ou veineux en identifiant les zones dans lesquelles l'intensité du signal de fluorescence diminue plus tardivement que dans d'autres.

Etape 8 : En outre, le logiciel permet de normaliser l'ensemble des images de fluorescence Iᵢ par un seuil défini et fixé à l'avance. Le logiciel colorise ensuite les images ainsi normalisées en utilisant une table de conversion des couleurs. Par exemple, cette table de conversion est la même que celle utilisée à l'étape 5 ci-dessus.

Le logiciel superpose chaque image ainsi normalisée avec l'image courante de fond I_{Ci} correspondante. Le résultat est affiché. Cet affichage permet de comparer des dynamiques de perfusion entre patients et de les caractériser localement sur l'image courante (obtenue dans le visible ou le proche infra-rouge par exemple). En effet, par exemple, le seuil peut être choisi de manière à correspondre à un niveau moyen standard d'un pied sain. Un affichage en fausses couleurs permet alors de visualiser rapidement si le niveau moyen d'intensité de la fluorescence mesuré pour un patient est standard, ou s'il est plus fort ou plus faible (voir figure 5).

On peut choisir par exemple la convention suivante : si les couleurs sont chaudes c'est que la zone est correctement perfusée par rapport à un niveau standard de perfusion. A l'inverse, des couleurs froides témoignent d'une vascularisation plus faible que la moyenne.

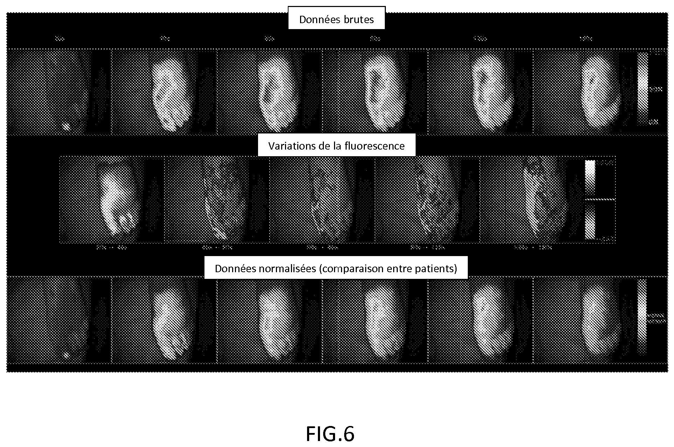

L'ensemble des images calculées peut être affiché simultanément (les figures 3, 4 et 5 sont affichées ensemble, par exemple de manière analogue à la figure 6).

Selon une variante, l'intervalle de temps *Δt* entre chaque séquence n'est pas régulier. Par exemple, cet intervalle de temps *Δt* peut être égal à vingt secondes, puis quarante secondes, puis soixante secondes. Un exemple de résultat obtenu avec des intervalles *Δt* variables est représenté sur la figure 6. Dans ce cas, avantageusement, la normalisation de l'image résultante doit prendre en compte cette variation des intervalles de temps.

Le procédé selon l'invention permet donc de faciliter l'interprétation des mesures d'un signal de fluorescence à l'aide d'une représentation visuelle, avec notamment une information précise des dynamiques locales des signaux de fluorescence, et en particulier les paramètres suivants :
- délai d'apparition du signal de fluorescence,
- variation de l'intensité du signal de fluorescence au cours du temps,
- localisation des montées et descentes du signal de fluorescence au cours du temps.

Le procédé selon l'invention permet des comparaisons entre patients.

Le procédé selon l'invention fournit un outil d'analyse automatique, notamment lors de son utilisation en normalisant par rapport à un seuil de référence (voir exemple du pied sain ci-dessus).sans intervention ou choix arbitraire (notamment en choisissant un mauvais tissu de référence) d'un praticien qui pourrait introduire des biais et donc des erreurs d'interprétation des résultats. Il permet aussi de voir rapidement les cas où il y a une erreur manifeste dans le calcul ce qui ne serait pas possible sur une courbe (par exemple aux bordures d'un pied si la scène a trop bougé et/ou qu'elle n'est ne peut plus être recalée, par exemple si le pied sort du champ de vue, ou si un objet passe dans le champ et entraine un artéfact de mesure). Cependant, les recalages d'images permettent de gérer facilement et efficacement des scènes qui peuvent bouger dans le temps (notamment le mouvement des pieds d'un patient). Il permet donc de travailler sur des tissus déformables et/ou qui peuvent bouger dans le temps. Il permet, grâce notamment à la détection des pixels saturés et aux normalisations des images, de gérer efficacement les surexpositions. Il permet grâce notamment à la combinaison d'images de fluorescence prises à des temps d'exposition ou des gains différents, de gérer efficacement les sous-expositions. Il permet une comparaison rapide des différentes zones d'un tissu et entre tissus de même type.

## Revendications

1. Procédé de suivi de la diffusion dans le temps d'un marqueur fluorescent au sein d'un tissu biologique (20), ce procédé comprenant :
- l'excitation du marqueur fluorescent à l'aide d'une source de lumière d'excitation,
- l'acquisition, à l'aide d'une caméra, d'images de fluorescence d'une zone du tissu biologique, chaque image de fluorescence correspondant à un ensemble de pixels, une valeur d'un signal représentatif de l'intensité de l'émission de fluorescence en un point de la zone du tissu biologique étant associée à chaque pixel,
- un traitement d'au moins une image de fluorescence, et
- l'affichage sur un écran (3) d'au moins une image résultant du traitement d'une image de fluorescence,
**Caractérisé par le fait**
- **que** l'acquisition des images de fluorescence comprend au moins deux séquences d'acquisition d'images, chacune de ces séquences commençant respectivement à des temps Tᵢ différents d'une même perfusion du marqueur fluorescent, avec i compris entre 0 et F, T₀ correspondant au moment auquel le marqueur fluorescent est injecté dans le tissu biologique et T_{F} correspondant au moment de la fin de l'acquisition des images de fluorescence,
- par le fait qu'il comprend une opération de comparaison de deux images résultant chacune respectivement du traitement d'images acquises au cours de deux séquences successives d'acquisition commençant respectivement à des temps Tᵢ différents, et
- par le fait qu'il comprend un affichage sur un écran du résultat de cette opération de comparaison sous forme d'une image représentative de la zone de tissu biologique.

2. Procédé selon la revendication 1, dans lequel l'opération de comparaison comprend au moins une opération choisie parmi les opérations suivantes : une soustraction entre des valeurs d'un signal représentatif de l'intensité de l'émission de fluorescence, un calcul de norme d'une grandeur représentée par des valeurs d'un signal représentatif de l'intensité de l'émission de fluorescence, un calcul de distance algébrique entre des valeurs d'un signal représentatif de l'intensité de l'émission de fluorescence, et une opération logique « or » ou « nor » ou « xor » sur des valeurs d'un signal représentatif de l'intensité de l'émission de fluorescence.

3. Procédé selon l'une des revendications précédentes, dans lequel un traitement des images de fluorescence est réalisé pour une même série d'images de fluorescence pour les recaler entre elles avant de les sommer pixel par pixel.

4. Procédé selon l'une des revendications précédentes, dans lequel une séquence d'acquisition d'images comporte l'acquisition d'une image courante de fond, cette image courante de fond étant utilisée lors de l'affichage des images de fluorescence, pour localiser les variations dynamiques du signal de fluorescence sur la zone de tissu biologique.

5. Procédé selon l'une des revendications précédentes, dans lequel chaque séquence d'acquisition d'image de fluorescence comprend l'acquisition d'au moins deux séries d'images de fluorescence, le temps d'exposition pouvant être différent pour chacune de ces séries d'images de fluorescence.

6. Dispositif de suivi de la fluorescence émise à la surface du tissu biologique (20), comprenant :
- une source d'excitation adaptée pour émettre un rayonnement d'excitation d'un marqueur de fluorescence,
- une caméra comprenant un capteur de la lumière de fluorescence émise à la surface du tissu biologique (20), sous l'effet du rayonnement d'excitation,
- un ordinateur (2) pour enregistrer et stocker des images de fluorescence capturées par la caméra, et pour réaliser un traitement des images de fluorescence, et
- un écran (3) pour afficher des images résultant du traitement des images de fluorescence par l'ordinateur,
l'ordinateur comprenant des moyens de calcul configurés pour traiter des images de fluorescence à l'aide
d'un logiciel pour la mise en œuvre du procédé selon l'une des revendications précédentes.

7. Dispositif selon la revendication 6, comprenant une source de lumière éclairant dans une bande spectrale excitant la fluorescence d'un marqueur et dans laquelle le capteur de la lumière de fluorescence n'est pas sensible.

## Patentansprüche

1. Verfahren zur Verfolgung der zeitlichen Diffusion eines fluoreszenten Markers in einem biologischen Gewebe (20), wobei dieses Verfahren umfasst:
- das Anregen des fluoreszenten Markers mithilfe einer Anregungslichtquelle,
- das Erfassen, mithilfe einer Kamera, von Fluoreszenzbildern eines Bereichs des biologischen Gewebes, wobei jedes Fluoreszenzbild einer Menge von Pixeln entspricht, wobei jedem Pixel ein Wert eines für die Intensität der Fluoreszenzemission in einem Punkt des Bereichs des biologischen Gewebes repräsentativen Signals zugeordnet ist,
- eine Verarbeitung mindestens eines Fluoreszenzbilds, und
- das Anzeigen mindestens eines aus der Verarbeitung eines Fluoreszenzbilds hervorgegangenen Bilds auf einem Bildschirm (3),
**Dadurch gekennzeichnet,**
- **dass** das Erfassen der Fluoreszenzbilder mindestens zwei Bilderfassungssequenzen umfasst, wobei jede dieser Sequenzen zu unterschiedlichen Zeiten Tᵢ einer selben Perfusion des fluoreszenten Markers mit i zwischen 0 und F beginnt, wobei T₀ dem Zeitpunkt entspricht, zu dem der fluoreszente Marker in das biologische Gewebe injiziert wird, und T_{F} dem Zeitpunkt des Endes der Erfassung der Fluoreszenzbilder entspricht,
- dadurch, dass es eine Operation zum Vergleichen von zwei Bildern umfasst, von denen jedes aus der Verarbeitung von Bildern hervorgegangen ist, die im Laufe von zwei aufeinander folgenden Erfassungssequenzen erfasst wurden, die jeweils zu unterschiedlichen Zeiten Tᵢ begonnen haben, und
- dadurch, dass es eine Anzeige des Ergebnisses dieser Vergleichsoperation in Form eines für den Bereich des biologischen Gewebes repräsentativen Bilds auf einem Bildschirm umfasst.

2. Verfahren nach Anspruch 1, wobei die Operation des Vergleichens mindestens eine Operation umfasst, die unter den folgenden Operationen gewählt ist: einer Subtraktion zwischen Werten eines für die Intensität der Fluoreszenzemission repräsentativen Signals, einer Normberechnung einer Größe, die durch Werte eines für die Intensität der Fluoreszenzemission repräsentativen Signals repräsentiert wird, einer Berechnung des algebraischen Abstands zwischen Werten eines für die Intensität der Fluoreszenzemission repräsentativen Signals und einer logischen "OR" oder "NOR" oder "XOR"-Operation an Werten eines für die Intensität der Fluoreszenzemission repräsentativen Signals.

3. Verfahren nach einem der vorhergehenden Ansprüche, wobei eine Verarbeitung der Fluoreszenzbilder für eine selbe Serie von Fluoreszenzbildern ausgeführt wird, um sie aufeinander zu registrieren, bevor sie Pixel für Pixel summiert werden.

4. Verfahren nach einem der vorhergehenden Ansprüche, wobei eine Bilderfassungssequenz das Erfassen eines aktuellen Hintergrundbilds beinhaltet, wobei dieses aktuelle Hintergrundbild beim Anzeigen der Fluoreszenzbilder verwendet wird, um die dynamischen Änderungen des Fluoreszenzsignals in dem Bereich des biologischen Gewebes zu lokalisieren.

5. Verfahren nach einem der vorhergehenden Ansprüche, wobei jede Fluoreszenzbilderfassungssequenz das Erfassen von mindestens zwei Fluoreszenzbildserien umfasst, wobei die Belichtungszeit bei jeder dieser Fluoreszenzbildserien unterschiedlich sein kann.

6. Vorrichtung zur Verfolgung der an der Oberfläche des biologischen Gewebes (20) emittierten Fluoreszenz, umfassend:
- eine Anregungsquelle, die dazu angepasst ist, eine Strahlung zur Anregung eines Fluoreszenzmarkers zu emittieren,
- eine Kamera, die einen Sensor für das an der Oberfläche des biologischen Gewebes (20) unter der Wirkung der Anregungsstrahlung emittierte Fluoreszenzlicht umfasst,
- einen Computer (2) zum Aufzeichnen und Speichern der von der Kamera aufgenommenen Fluoreszenzbilder und zum Ausführen einer Verarbeitung der Fluoreszenzbilder, und
- einen Bildschirm (3) zum Anzeigen der aus der Verarbeitung der Fluoreszenzbilder durch den Computer hervorgegangenen Bilder,
wobei der Computer Rechenmittel umfasst, die dazu ausgelegt sind, Fluoreszenzbilder mithilfe einer Software zur Durchführung des Verfahrens nach einem der vorhergehenden Ansprüche zu verarbeiten.

7. Vorrichtung nach Anspruch 6, die eine Lichtquelle umfasst, die in einem Spektralband beleuchtet, das die Fluoreszenz eines Markers anregt, und bei welcher der Sensor für das Fluoreszenzlicht nicht empfindlich ist.

## Claims

1. Method for monitoring the diffusion over time of a fluorescent marker within a biological tissue (20), this method comprising:
- exciting the fluorescent marker using an excitation light source,
- acquiring, using a camera, fluorescence images of an area of the biological tissue, each fluorescence image corresponding to a set of pixels, a value of a signal representative of the intensity of the emission of fluorescence at a point in the area of the biological tissue being associated with each pixel,
- processing at least one fluorescence image, and
- displaying on a screen (3) at least one image resulting from the processing of a fluorescence image,
**characterized in that**
- the acquisition of the fluorescence images comprises at least two image acquisition sequences, each of these sequences starting respectively at different times Tᵢ during a given perfusion of the fluorescent marker, with i comprised between 0 and F, T₀ corresponding to the time at which the fluorescent marker is injected into the biological tissue and T_{F} corresponding to the time at which the acquisition of the fluorescence images ends,
- **in that** it comprises an operation of comparing two images each respectively resulting from the processing of images acquired during two successive acquisition sequences respectively starting at different times Tᵢ, and
- **in that** it comprises displaying on a screen the result of this comparing operation in the form of an image representative of the area of biological tissue.

2. Method according to Claim 1, wherein the comparing operation comprises at least one operation chosen from the following operations: a subtraction between values of a signal representative of the intensity of the fluorescence emission, a computation of a norm of a quantity represented by values of a signal representative of the intensity of the fluorescence emission, a computation of an algebraic distance between values of a signal representative of the intensity of the fluorescence emission, and an "or" or "nor" or "xor" logic operation on values of a signal representative of the intensity of the fluorescence emission.

3. Method according to one of the preceding claims, wherein processing of the fluorescence images is carried out for a given series of fluorescence images in order to align them with one another before summing them pixel by pixel.

4. Method according to one of the preceding claims, wherein an image acquisition sequence comprises acquiring a current background image, this current background image being used, when displaying the fluorescence images, to locate dynamic variations in the fluorescence signal in the area of biological tissue.

5. Method according to one of the preceding claims, wherein each fluorescence image acquisition sequence comprises acquiring at least two series of fluorescence images, the exposure time possibly being different for each of these series of fluorescence images.

6. Device for monitoring the fluorescence emitted from the surface of the biological tissue (20), comprising:
- an excitation source suitable for emitting excitation radiation in order to excite a fluorescence marker,
- a camera comprising a sensor of the fluorescence light emitted from the surface of the biological tissue (20), under the effect of the excitation radiation,
- a computer (2) for recording and storing fluorescence images captured by the camera, and for processing the fluorescence images, and
- a screen (3) for displaying images resulting from the processing of the fluorescence images by the computer,
the computer comprising computing means configured for processing fluorescence images using a software package for implementing the method according to one of the preceding claims.

7. Device according to Claim 6, comprising a light source that generates illumination in a spectral band that excites the fluorescence of a marker but to which the fluorescence light sensor is not sensitive.
